# EUROPEAN PATENT SPECIFICATION

(11) **EP 4 244 346 B1**
(45) Date of publication and mention of the grant of the patent: **08.07.2026**
(21) Application number: 21892952.9
(22) Date of filing: 14.11.2021
(51) Int. Cl.: C12N 9/24, A61K 38/47, A61P 31/04, A01N 63/50, C12N 15/62

(54) **ENGINEERED CHIMERIC ANTIMICROBIAL AGENTS AGAINST GRAM-POSITIVE BACTERIA**
MANIPULIERTE CHIMÄRE ANTIMIKROBIELLE MITTEL GEGEN GRAM-POSITIVE BAKTERIEN
AGENTS ANTIMICROBIENS CHIMÉRIQUES MODIFIÉS CONTRE DES BACTÉRIES À GRAM POSITIF

(30) Priority: 13.11.2020 SG 10202011321V
(43) Date of publication of application: 20.09.2023
(73) Proprietor: Massachusetts Institute of Technology, Cambridge, MA 02139 (US)
(72) Inventor: GOH, Boon, Chong, Singapore 138602 (SG)
(74) Representative: Mewburn Ellis LLP
(86) International application number: PCT/US2021/059290
(87) International publication number: WO 2022/104171

(56) References cited:
- WO-A1-2013/169104
- WO-A2-2017/049242
- VERBREE CAROLIN T. ET AL: "Corrected and Republished from: Identification of Peptidoglycan Hydrolase Constructs with Synergistic Staphylolytic Activity in Cow's Milk", vol. 84, no. 1, 1 January 2018 (2018-01-01), US, XP055881646, ISSN: 0099-2240, Retrieved from the Internet <URL:https://journals.asm.org/doi/pdf/10.1128/AEM.02134-17> DOI: 10.1128/AEM.02134-17
- HORGAN MARIANNE ET AL: "Phage Lysin LysK Can Be Truncated to Its CHAP Domain and Retain Lytic Activity against Live Antibiotic-Resistant Staphylococci", APPLIED AND ENVIRONMENTAL MICROBIOLOGY, AMERICAN SOCIETY FOR MICROBIOLOGY, US, vol. 75, no. 3, 1 February 2009 (2009-02-01), pages 872 - 874, XP002574789, ISSN: 0099-2240, DOI: 10.1128/AEM.01831-08
- DONG Q. ET AL.: "Construction of a chimeric lysin Ply187N-V12C with extended lytic activity against staphylococci and streptococci", MICROB BIOTECHNOL, vol. 8, no. 2, 15 September 2014 (2014-09-15), pages 210 - 220, XP055433293, [retrieved on 20220318], DOI: 10.1111/1751-7915.12166>
- HUANG L. ET AL.: "Construction and characterization of a chimeric lysin ClyV with improved bactericidal activity against Streptococcus agalactiae in vitro and in vivo", APPL MICROBIOL BIOTECHNOL, vol. 104, no. 4, 3 January 2020 (2020-01-03), pages 1609 - 1619, XP036998575, [retrieved on 20220318], DOI: 10.1007/S00253-019-10325-Z
- CHENG MENGJUN, ZHANG YUFENG, LI XINWEI, LIANG JIAMING, HU LIYUAN, GONG PENGJUAN, ZHANG LEI, CAI RUOPENG, ZHANG HAO, GE JINLI, JI Y: "Endolysin LysEF-P10 shows potential as an alternative treatment strategy for multidrug-resistant Enterococcus faecalis infections", SCIENTIFIC REPORTS, vol. 7, no. 1, 1 December 2017 (2017-12-01), XP055939026, DOI: 10.1038/s41598-017-10755-7
- DANIEL A. ET AL.: "Synergism between a novel chimeric lysin and oxacillin protects against infection by methicillin-resistant Staphylococcus aureus", ANTIMICROB AGENTS CHEMOTHER, vol. 54, no. 4, 19 January 2010 (2010-01-19), pages 1603 - 1612, XP055019034, [retrieved on 20220318], DOI: 10.1128/AAC.01625-09
- YANG H. ET AL.: "Novel chimeric lysin with high-level antimicrobial activity against methicillin-resistant Staphylococcus aureus in vitro and in vivo", ANTIMICROB AGENTS CHEMOTHER, vol. 58, no. 1, 4 November 2013 (2013-11-04), pages 536 - 542, XP055434200, [retrieved on 20220318], DOI: 10.1128/AAC.01793-13
- BINTE MUHAMMAD JAI HANA SAKINA, DAM LINH CHI, TAY LOWELLA SERVITO, KOH JODI JIA WEI, LOO HOOI LINN, KLINE KIMBERLY A., GOH BOON CH: "Engineered Lysins With Customized Lytic Activities Against Enterococci and Staphylococci", FRONTIERS IN MICROBIOLOGY, vol. 11, 25 November 2020 (2020-11-25), XP055939029, DOI: 10.3389/fmicb.2020.574739

## Description

### FIELD OF THE INVENTION

The present disclosure relates to chimeric bacteriophage lysins which can be used to sanitize surfaces, for the treatment of bacterial infections and/or for the selective treatment of body odor-causing bacteria. The disclosure also includes synergistic combinations, isolated polynucleotides which encode the chimeric lysins.

### BACKGROUND OF THE INVENTION

Antimicrobial resistance (AMR) is on the rise worldwide, which poses a public health crisis given the slow pace of antibiotic development. To treat infections caused by drug-resistant bacteria, health care providers are resorting to mothballed antibiotics that come with serious side effects [Velez, R. and Sloand, E., J Clin Nurs 25: 1886-1889 (2016); Ventola, C. L. P T 40: 277-83 (2015)]. Therefore, there is a dire need to develop novel antibacterial agents. Lysins derived from bacteriophage present a promising alternative to antibiotics as they are less prone to resistance and can kill bacteria selectively and rapidly [Fischetti, V. A., Curr Opin Microbiol 11: 393-400 (2008); Fischetti, V. A., Viruses 10 (2018); Schmelcher, M. et al., Future Microbiol 7: 1147-1171 (2012); Zou, G. et al., J Biol Chem 286: 14362-14372 (2011); Maciejewska, B. et al., Appl Microbiol Biotechnol 102: 2563-2581 (2018); Oliveira, H. et al., Viruses 10 (2018)].

Lysins are phage-encoded peptidoglycan hydrolases that cleave the bonds in peptidoglycan chains of the bacteria outer cell wall. When applied exogenously to bacteria as purified recombinant proteins, lysins rapidly hydrolyze the peptidoglycan layer, resulting in cell lysis and bacterial death. Hence, lysins are particularly effective against Gram-positive bacteria because of the thick peptidoglycan layer on the bacterial surface [Fischetti, V. A., Curr Opin Microbiol 11: 393-400 (2008); Hermoso, J. A. et al., Curr Opin Microbiol 101-472 (2007); Fischetti, V. A. Int J Med Microbiol 300: 357-362 (2010)]. Lysins targeting Gram-positive bacteria contains two functional, modular domains, namely catalytic domain (CD) and cell wall binding domain (CBD). CD cleaves the specific bonds of the bacterial peptidoglycan, and CBD recognizes the target bacteria and anchors the lysin by binding to specific carbohydrate ligands on the bacterial cell wall, contributing to the selective nature of lysine [Loessner, M. J. et al., Mol Microbiol 44: 335-349 (2002); Lopez, R. et al., Microb Drug Resist 3: 199-211 (1997)].

The lytic spectrum of a lysin ranges from species-specific to targeting multiple genera. There are lysins that specifically target a single bacterial species (e.g., LysEF-P10 for *E*. *faecalis* [Cheng, M. et al., Sci Rep 7: 1-15 (2017)], and PlyPSA for *Listeria monocytogenes* [Schmelcher, M. et al., Microb Biotechnol 4: 651-662 (2011); Zimmer, M. et al., Mol Microbiol 50: 303-317 (2003)]) while some target various bacterial species of the same genus (e.g. LysK [O'Flaherty, S. et al., J Bacteriol 187: 7161-7164 (2005)]). Interestingly, several lysins have been identified to target multiple genera [Deutsch, S.-M. et al., Appl Environ Microbiol 70: 96-103 (2004); Yoong, P. et al., J Bacteriol 186: 4808-4812 (2004); Gilmer, D. B. et al., Antimicrob Agents Chemother 57: 2743-2759 (2013)]. For example, PlyV12 targets enterococci, staphylococci, and streptococci [Yoong, P. et al., J Bacteriol 186: 4808-4812 (2004)], and PlySs2 lyses staphylococci, streptococci and listeria [Gilmer, D. B. et al., Antimicrob Agets Chemother 57: 2743-2759 (2013)].

The modular architecture of Gram-positive lysin enables the construction of chimeric lysins with enhanced lytic spectrum and lytic activity [Loessner, M. J. et al., Mol Microbiol 44: 335-349 (2002); Schmelcher, M. et al., Microb Biotechnol 4: 651-662 (2011); Yang, H. et al., Front Microbiol 5: 1- (2014); Dong, Q. et al., Microb Biotechnol 8: 210-220 (2015); Broendum, S. S. et al., Mol Microbiol 110: 879-896 (2018)]. For example, a chimeric lysin Ply187N-V12C exhibited a broader lytic spectrum from only targeting staphylococci to also kill enterococci and streptococci after adding the CBD of PlyV12 [Dong, Q. et al., Microb Biotechnol 8: 210-220 (2015)]. While the CBD largely determines the lytic spectrum by recognizing specific cell wall components on the bacteria, the CD dictates the type of peptidoglycan bond to be cleaved [Broendum, S. S. et al., Mol Microbiol 110: 879-896 (2018); Oliveira, H. et al., J Virol 87: 4558-4570 (2013); Nelson, D. C. et al., Advances in Virus Research (2012); Diez-Martinez, R. et al., Antimicrob Agents Chemother 57: 5355-5365 (2013)]. The dependence of CBD for target recognition can be circumvented by tweaking the net charge of a lysin. A positively-charged CD-only lysin was shown to maintain its lytic activity while possessing a broader lytic spectrum [Low, L. Y. et al., J Biol Chem 286: 34391-34403 (2011)]. Bactericidal activity can be improved by fusing the CBD of a streptococcal lysin with the CD of a different lysin, or by modifying the linker between CD and CBD [Yang, H. et al., Antimicrob Agents Chemother 63 (2019); Yang, H. et al., Antimicrob Agents Chemother 64 (2020)].

While there are many reports that successfully extended the lytic spectrum of a lysin [Schmelcher, M. et al., Microb Biotechnol 4: 651-662 (2011); Dong, Q. et al., Microb Biotechnol 8: 210-220 (2015); Broendum, S. S. et al., Mol Microbiol 110: 896 (2018); Diez-Martinez, R. et al., Antimicrob Agents Chemother 57: 5355-5365 (2013)], few have managed to introduce species specificity in a chimeric lysin.

A promising application of lysins is on the human skin, particularly to eradicate the problem of body odor (BO). The formation of body odors (BO) is caused by factors such as diet, gender, health, and medication, but the major contribution comes from bacterial activity on skin gland secretions. *Staphylococcus hominis, Staphylococcus haemolyticus, Staphylococcus epidermidis,* and *Corynebacterium jeikeium* are often associated with producing BO in human skin, particularly in the armpits. BO gases are produced when these BO-causing bacteria degrade the amino acids and fatty acids found in sweat.

Current anti-BO solutions in the market can be categorized into two types: antiperspirant, which reduces sweat production, and deodorant, which masks odor. None of the existing solutions target the underlying cause of BO, i.e., bacteria. WO2013/169104 A1 discloses polypeptide mixes with antibacterial activity. Verbree et al., Appl Environ Microbiol. 2017; 84(1) discloses peptidoglycan hydrolase constructs with synergistic staphylolytic activity in cow's milk. Horgan et al., Environ Microbiol. 2009;75(3):872-874 discloses that phage lysin LysK can be truncated to its CHAP domain and retain lytic activity against live antibiotic-resistant staphylococci. WO2017/049242 A2 discloses uses of lysin to restore/augment antibacterial activity in the presence of pulmonary surfactant of antibiotics inhibited thereby. Dong et al. Microb Biotechnol. 2014;8(2):210-220 discloses a chimeric lysin Ply187N-V12C with extended lytic activity against staphylococci and streptococci. Muhammad et al. Front Microbiol. 2020 Nov 25;11:574739 discloses engineered lysins with customized lytic activities against enterococci and staphylococci.

There is a need for alternative solutions that target problematic bacteria.

### SUMMARY OF THE INVENTION

Chimeric lysins were created by interchanging the CBDs of parent lysins. The lytic spectra of these chimeric lysins were characterized by subjecting them to a panel of enterococcal and staphylococcal strains under various conditions. Certain chimeric lysins are useful for various infections, whereas particular chimeric lysins have been developed for a new treatment to selectively eliminate BO-causing bacteria. The key advantages of lysins include their good safety profile, as lysins are not systemically absorbed, and are biodegradable.

In a first aspect the invention provides a chimeric bacteriophage lysin comprising a catalytic domain of a first phage lysin and a binding domain of a second phage lysin, wherein the chimeric bacteriophage lysin has killing activity against a plurality of *Staphylococcus* species, wherein the chimeric bacteriophage lysin comprises an amino acid sequence set forth in:
amino acids 1-165 and 170-338 set forth in SEQ ID NO: 9, or a variant having at least 90% identity to the amino acid sequence set forth in SEQ ID NO: 9,
and wherein the chimeric bacteriophage lysin has killing activity against *S. hominis* and
*S. epidermidis* in human sweat.

In some embodiments the chimeric bacteriophage lysin variant comprises a sequence having at least 95% sequence identity to SEQ ID NO: 9.

In some embodiments, the said catalytic domain of a first phage lysin and binding domain of a second phage lysin are fused by a linker peptide.

In some embodiments, the linker peptide has the amino acid sequence set forth in amino acids 166-169 of SEQ ID NO: 9.

In some embodiments the chimeric bacteriophage lysin has the amino acid sequence set forth in SEQ ID NO: 9;

In some embodiments the chimeric bacteriophage lysin has killing activity against *S. hominis* and *S. epidermidis* in human sweat.

Also disclosed is a chimeric lysin that has killing activity against methicillin-resistant and methicillin-sensitive strains of *S*. *aureus.*

Also disclosed is a chimeric lysin that has killing activity against Rifampicin resistant strains of *E. faecalis* and Vancomycin resistant strains of *E. faecalis.*

Also disclosed is a chimeric lysin that has killing activity over a pH range of about 4-10 and/or over a salt concentration of about 0-500 mM NaCl.

In a second aspect, the invention provides an isolated polynucleotide molecule encoding a chimeric bacteriophage lysin defined in the first aspect.

In some embodiments of the isolated polynucleotide molecule the nucleic acid sequence has at least 85% sequence identity to SEQ ID NO: 10 due to the degeneracy of the genetic code.

In a third aspect, the invention provides a composition comprising one or more chimeric bacteriophage lysins of the first aspect. Preliminary studies have shown potential synergistic effect by using two different lysins. It has also been found that a combination of a chimeric bacteriophage lysin such as LKCHAPV12 (having the amino acid sequence set forth in SEQ ID NO: 9) and native lysin such as PlyV12 (having the amino acid sequence set forth in SEQ ID NO: 17, and the nucleic acid sequence set forth in SEQ ID NO: 18) can act synergistically in killing Staphylococci (Table 4). LysEF-P10 (having the amino acid sequence set forth in SEQ ID NO: 19, and the nucleic acid sequence set forth in SEQ ID NO: 20) is a native lysin that may be combined with a chimeric bacteriophage lysin for synergistic effect.

In some embodiments, the composition is a synergistic composition comprising one or more chimeric bacteriophage lysins and one or more native lysins.

In some embodiments, the composition is for sanitizing or decontaminating porous or non-porous surfaces comprising at least one chimeric bacteriophage lysin of any aspect of the invention or the composition of the third aspect.

In some embodiments, the composition is for medical or cosmetic use.

In some embodiments the composition further comprises an antibiotic.

In some embodiments, the composition is formulated for administration to the skin. Suitable formulations would be well-known to those skilled in the art. Formulations may include chimeric bacteriophage lysins of any aspect of the invention encapsulated within lipid/polymer nano or microparticles.

The disclosure provides use of a composition of the third aspect for the manufacture of a medicament for the prophylaxis or treatment of a staphylococcal infection.

In some examples, the medicament is for the prophylaxis or treatment of body odor.

In some examples, the medicament comprises at least one chimeric lysin selected from the group comprising the chimeric lysin defined in the first aspect.

In some examples, the medicament is for the prophylaxis or treatment of an *E*. *faecalis* infection.

The disclosure provides a method of prophylaxis or treatment of a staphylococcal infection in a mammal, comprising administering to said mammal one or more chimeric lysins of the first aspect or a composition of the third aspect.

In some examples, the staphylococcal infection causes body odor in a human.

In a further aspect, the invention provides a method for decontaminating inanimate surfaces suspected of containing infectious bacteria, comprising treatment of said surfaces with a bactericidally or bacteriostatically effective amount of at least one chimeric bacteriophage lysin of any aspect of the invention or the composition of the third aspect.

### BRIEF DESCRIPTION OF THE FIGURES

**Figure 1** shows a description of the parent and chimeric recombinant lysins. (A) Schematic diagram illustrating the domain swapping between the parent lysins to create the two chimeras. The N-terminal domain is the catalytic domain whereas the C-terminal domain is the cell-wall binding domain. (B) The SDS-PAGE analysis showed the high purity of the produced lysins. PlyV12 (35.0 kDa), P10N-V12C (35.4 kDa), V12N-P10C (27.2 kDa) contain a C-terminal 6xHis tag, while LysEF-P10 (29.1 kDa) contains a N-terminal 6xHis-TEV for affinity chromatography purification.
**Figure 2** shows lytic activity characterization. (A) Killing kinetics of PlyV12 against *E*. *faecalis* OR1GF strain at various doses as measured by the reduction in OD₆₀₀. (B) Lytic activity at various doses as measured by the rate of reduction in OD₆₀₀ in the first 3 minutes. These experiments were performed in biological triplicates.
**Figure 3** shows the dose response for other 3 lysins. Lysin concentrations that achieved >90% OD₆₀₀ reduction were used in this study.
**Figure 4** shows characterization of lysins. The lytic activities of the four lysins at various pH buffers (A) under different salt concentrations (B). These experiments were done in biological triplicates.
**Figure 5** shows lytic spectra of the parent and chimeric lysins. The activities of these four lysins against 12 enterococcal and 10 staphylococcal strains were measured as the reduction in OD₆₀₀ per minute in the first three minutes. These experiments were done in biological triplicates.

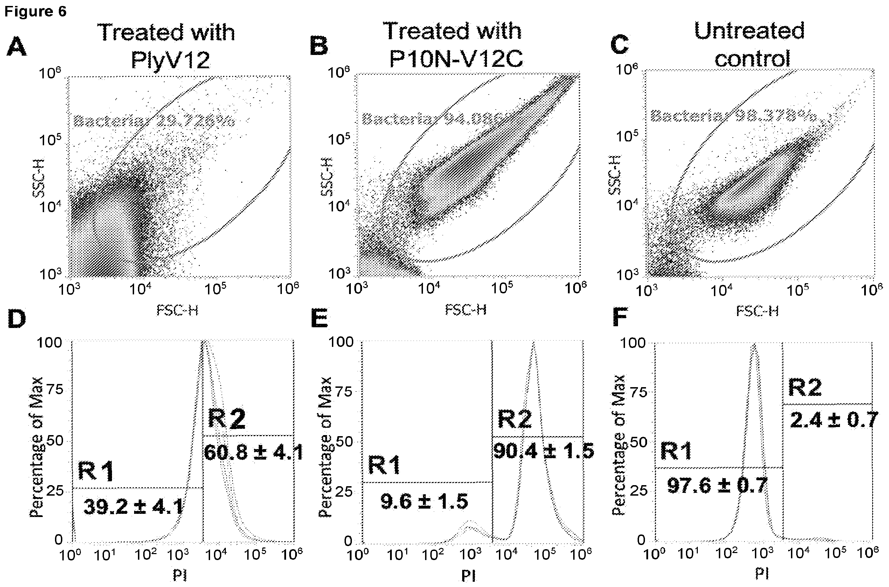

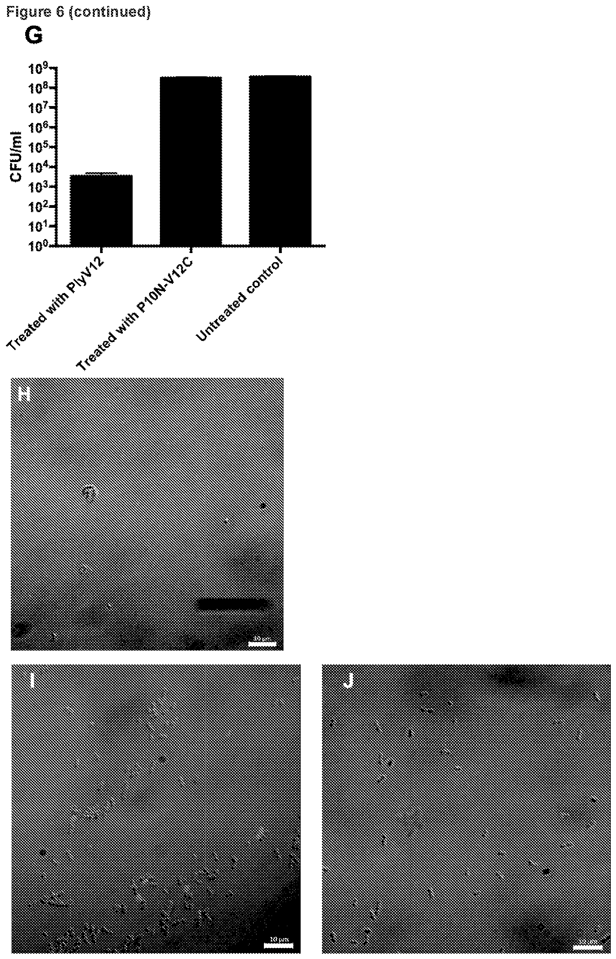

**Figure 6** shows the effect of lysin treatment on *E. faecium.* FSC-SSC dot plots of *E*. *faecium* cells upon treated with PlyV12 (A), P10N-V12C (B), and without lysin treatment (C). (D-F) The corresponding histogram displaying percentage of gated cells with PI uptake. PI-negative and Pl-positive cells are labeled with R1 and R2 respectively. (G) The bactericidal activity of the two lysins on *E. faecium.* Confocal microscopy images of the *E. faecium* cells with treatment of PlyV12 (H), P10N-V12C (I) and without lysin treatment (J). Cells with PI uptake were detected as red fluorescence signals. White bars indicate 10 µm.
**Figure 7** shows controls for the flow cytometry experiments used to establish gating parameters. The FSC-SSC plots are at the top row, whereas the bottom row shows the percentage of gated cells with PI uptake. PI-negative and Pl-positive cells are labeled with R1 and R2 respectively.
**Figure 8** shows SSC-FSC plots of the 4 replicates that were performed. PlyV12 and P10N-V12C lysin treated E faecium cells are shown in the top and middle rows, respectively. The untreated control runs are shown in the bottom row. BR1(1) refers to biological replicate 1, technical duplicate 1.

### DETAILED DESCRIPTION OF THE INVENTION

### Definitions

Certain terms employed in the specification, examples and appended claims are collected here for convenience.

As used in the specification and the appended claims, the singular forms "a," "an" and "the" include plural referents unless the context clearly dictates otherwise.

The terms "amino acid" or "amino acid sequence," as used herein, refer to an oligopeptide, peptide, polypeptide, or protein sequence, or a fragment of any of these, and to naturally occurring or synthetic molecules. Where "amino acid sequence" is recited herein to refer to an amino acid sequence of a naturally occurring protein molecule, "amino acid sequence" and like terms are not meant to limit the amino acid sequence to the complete native amino acid sequence associated with the recited protein molecule.

The term "variant" as used herein, refers to an amino acid sequence that is altered by one or more amino acids, but retains the ability of the non-variant reference sequence to bind to and kill staphylococci. The variant may have "conservative" changes, wherein a substituted amino acid has similar structural or chemical properties (e.g., replacement of leucine with isoleucine). More rarely, a variant may have "non-conservative" changes (e.g., replacement of glycine with tryptophan). Analogous minor variations may also include amino acid deletions or insertions, or both. Moreover, guidance in determining which amino acid residues may be substituted, inserted, or deleted without abolishing biological activity may be found using computer programs well known in the art, for example, DNASTAR^{®} software (DNASTAR, Inc. Madison, Wisconsin, USA). A non-limiting example in the context of chimeric lysins of the invention is the presence of a peptide linker between the catalytic domain and the binding domain. In some disclosures the chimeric bacteriophage lysin variant comprises a sequence having at least 85%, at least 90% or at least 95% sequence identity to SEQ ID NO: 11, SEQ ID NO: 9, SEQ ID NO: 13, or SEQ ID NO: 15.

The phrases "nucleic acid" or "nucleic acid sequence," as used herein, refer to an oligonucleotide, nucleotide, polynucleotide, or any fragment thereof, to DNA or RNA of genomic or synthetic origin which may be single-stranded or double-stranded and may represent the sense or the antisense strand, to peptide nucleic acid (PNA), or to any DNA-like or RNA-like material.

As used herein, the term "comprising" or "including" is to be interpreted as specifying the presence of the stated features, integers, steps or components as referred to, but does not preclude the presence or addition of one or more features, integers, steps or components, or groups thereof. However, in context with the present disclosure, the term "comprising" or "including" also includes "consisting of". The variations of the word "comprising", such as "comprise" and "comprises", and "including", such as "include" and "includes", have correspondingly varied meanings.

Bibliographic references mentioned in the present specification are for convenience listed in the form of a list of references and added at the end of the examples.

Having now generally described the invention, the same will be more readily understood through reference to the following examples which are provided by way of illustration, and are not intended to be limiting of the present invention.

### EXAMPLES

Standard molecular biology techniques known in the art and not specifically described were generally followed as described in Green and Sambrook, Molecular Cloning: A Laboratory Manual, Cold Springs Harbor Laboratory, New York (2012).

### Example 1

### Materials and Methods

### Cloning, expression and purification of phage lysins

Lysin genes PlyV12 (Genbank accession No. AAT01859.1) and LysEF-P10 (GenBank accession No. AQT27695.1) were synthesized and cloned into pNIC-CH and pNIC28-Bsa4 vectors by Bio Basic Inc. The nucleotide sequences were codon-optimized to improve the efficiency of soluble expression in *E. coli.* Chimeric lysin genes of P10N-V12C (SEQ ID NOs: 11 and 12) and V12N-P10C were produced using overlap extension PCR using the primers listed in Table 1.

**Table 1: Primer designs to create chimeric lysins P10N-V12C and V12N-P10C**

| Target gene | Primer | Sequence (5' to 3') | SEQ ID NO: |
|---|---|---|---|
| *PlyV12 insert* | plyV12 fwd | CTGAACGGTGGTTCTACCCC | 1 |
| | plyV12 rev | TTTGAAGGTACCCCACGCTTC | 2 |
| *PlyV12 chimeric* | P10N- | CGTCCGCCGTACGAAAAAGATACCCCGCTG | 3 |
| *insert* | V12C fwd | AACGGTGGTTCTACCCC | |
| | P10N-V12C rev | GGATCCTCAATGGTGGTGATGATGGTGCGC TTTGAAGGTACCCCACGCTTC | 4 |
| *LysEF-P10 insert* | LysEF-P10 fwd | GCGCCGAAACCGAAGG | 5 |
| | LysEF-P10 rev | CGCAACTTTGAACTGCGGGTGAGACAG | 6 |
| *LysEF-P10 chimeric insert* | V12N-P10C fwd | GTATAGCATGGGTTGGTACGTTTATCGTCTG GCGCCGAAACCGCCG | 7 |
| | V12N-P10C rev | GGATCCTCAATGGTGGTGATGATGGTGCGC AACTTTGAACTGCGGGTGAGACA | 8 |

To produce the 2 chimeric lysins, the C-terminal cell wall binding domain (CBD) of PlyV12 and LysEF-P10 was amplified as inserts for P10N-V12C and V12N-P10C respectively. The inserts were purified and extended using primers containing the sequences of the N-terminal CD and the C-terminal histag in the vector pNIC-CH. The extended PCR products will be used as megaprimers to swap the CBD of PlyV12 and LysEF-P10, creating the two chimeric lysins. After confirmed by sequencing, the plasmids were transformed into competent cells of BL21(DE3)-T1R *E. coli* Rosetta strain for efficient protein expression.

To produce the recombinant lysins, the transformed *E. coli* was incubated at 37°C in LB broth containing 34 µg/ml chloramphenicol and 50 µg/ml kanamycin until the culture reaches mid-log phase (OD₆₀₀ 0.5-0.6). After cooling the culture down, protein overexpression was induced by adding 0.2 mM isopropyl β-D-thiogalactoside and incubated at 16°C for 16-18 hours. Cells were harvested by centrifugation at 4,000 g for 25 min at 4 °C and the cell pellet was stored at -80°C until ready to purify.

To purify the proteins, the cell pellets were resuspended with lysis buffer before being disrupted by sonication on ice. PlyV12, chimeric lysins P10N-V12C and V12N-P10C used lysis buffer at pH 7.5 (50 mM HEPES pH 7.5, 500 mM NaCl, 5% glycerol, 0.5 mM DTT), whereas LysEF-P10 used lysis buffer at pH 8.5 (50 mM Tris pH 8.5, 500 mM NaCl, 5% glycerol, 0.5 mM DTT). Cell debris was removed by centrifugation for 1 hour at 40,000 x g at 4 °C to obtain the soluble protein. The supernatant was sterile filtered and mixed with 2 mL of nickel-nitrilotriacetic acid (Ni-NTA) resin and was incubated for 2 hours at 4°C in a tube rotator. The desired proteins were collected by washing and eluting with lysis buffers containing 0, 10, 20, 250, or 500, mM imidazole. Finally, the collected fractions were subjected to buffer exchange to reduce the imidazole concentration to <10mM and concentrated to >5 mg/ml before storing at -80 °C.

### Characterization of lytic activities and spectra of recombinant lysins.

Turbidity reduction assay was performed in 96-well microtiter plate to measure the lytic activity of the lysins produced [Nelson, D. et al., Proc Natl Acad Sci 98: 4107-4112 (2001); Yang, H. et al., Antimicrob Agents Chemother 58: 536-542 (2014)]. Enterococcal and staphylococcal strains were grown to mid-logarithmic phase in brain heart infusion (BHI) and Mueller Hilton (MH) broths, respectively. The bacteria were then pelleted and resuspended in assay buffers to an OD₆₀₀ of 1.0-1.2. Lysin was added to the bacteria with a volume ratio of 1:4 to a final volume of 100 µl. For dose response assays, 10 lysin concentrations of 0.78 to 400 µg/ml were tested. Upon identifying the optimal dose, 25 µg/ml of PlyV12 and 100 µg/ml of LysEF-P10, P10N-V12C, and V12N-P10C were used in the turbidity reduction assays for profiling their optimal pH and salt concentrations. The pH profile experiments were performed after the bacterial cells were resuspended in 20 mM acetate buffer at pH 5 and 6; 20 mM of phosphate buffer at pH 6, 7 and 8; and 20 mM Tris buffer at pH 9 and 10. The activities of the 4 lysins were also characterized under NaCl concentrations ranging from 0 mM to 500 mM. The lytic activity is measured by the reduction in OD₆₀₀ per minute for the first 3 minutes [Yang, H. et al., Sci Rep 7: 1-13 (2017)]. All assays were done with three biological replicates and all buffers were autoclaved before use. The bacterial OD₆₀₀ was measured in a microplate reader (Biotek Synergy 4 Plate Reader) for an hour with occasional shaking at 40-second intervals. Lysin-treated bacteria were serially diluted and plated on BHI and MH agar accordingly for enumeration of colony-forming units (CFU). As a negative control, bacterial strains were treated with equivalent quantity of buffer solutions.

### Flow Cytometry analysis of lysin-treated bacterial cells

*E. faecium* strain C5 was grown to mid-logarithmic phase (OD₆₀₀ = 0.5) in BHI. The bacteria were then pelleted and resuspended in 10% BHI diluted with 1X PBS, to an OD₆₀₀ of 0.08. Lysins PlyV12 and P10N-V12C were added to the resuspended bacterial cell on a 1:1 ratio such that the starting OD is 0.04 and the final concentrations of lysins are 25 µg/ml (PlyV12) and 100 µg/ml (P10N-V12C), respectively. After 1-hour incubation, 2 µg/ml of Propidium Iodide (PI) was added and incubated in the dark for 15 minutes at room temperature.

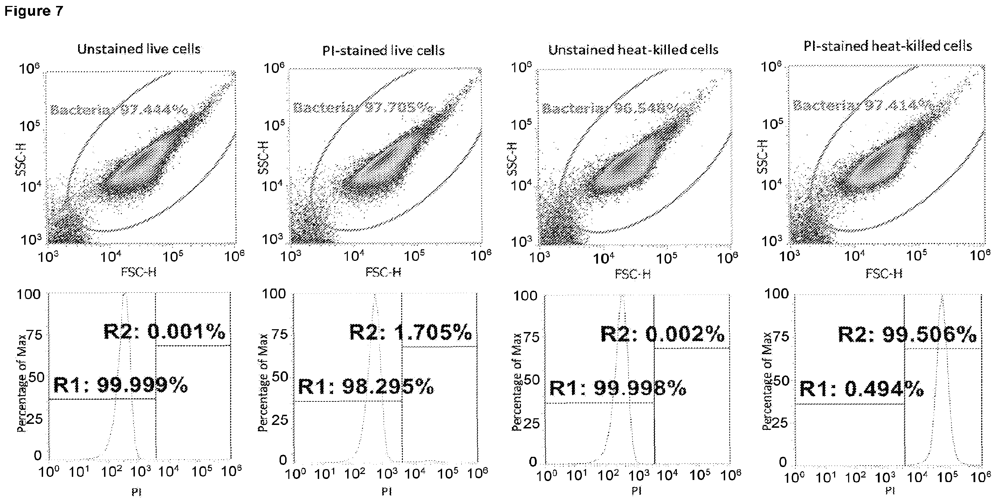

Flow cytometry analysis were performed using Attune NxT 4 lasers and 14 colors system, flat top laser from Thermo Fisher Scientific. Sample delivery utilizing positive-displacement syringe pump for direct volumetric analysis provides concentration measurement without using counting beads. The 96-well U-bottom sample plate was loaded and run with Attune NxT autosampler without prior washing. Total sample volume was 105 µl and acquisition volume was 40 µl. Data acquisition were set at flow rate of 25 µl/min with 2 mixing cycles and 3 rinsing cycles. Total events acquired were 100,000. 25 µl/min speed was the acquisition guideline provided by Thermo Fisher Scientific, with maximum sample concentration of not more than 5.4 x 10⁷/ml. PI was excited by 561 nm yellow Laser and fluorescent emission were detected at 620/15 band pass emission filter at YL2 PMT detector. The threshold was set at FSC (0.8 x1000) and SSC (1.0 x1000). Data were collected and analyzed using Attune NxT software. FSC and SSC voltage were set based on unstained healthy bacteria sample. Heat killed bacteria samples were used as a positive control for PI single staining (Fig. 7).

### Bacterial visualization using confocal microscopy.

Lysin-treated and untreated *E. faecium* cells were imaged using confocal laser scanning microscopy (CLSM). Propidium Iodine (PI) was added to all sample at a final concentration of 2 µg/mL for 15 minutes in the dark, similar to the flow cytometry experiments. Bacterial cells that are dead or have compromised membrane will uptake PI and appear red under CLSM. Confocal images were acquired on a Zeiss LSM700 CLSM system under bright field light and fluorescence using 40x and 63x oil-immersion objectives. Images were then processed using ZEN software (version 14.0).

### Example 2

### Construct design and expression of recombinant lysins

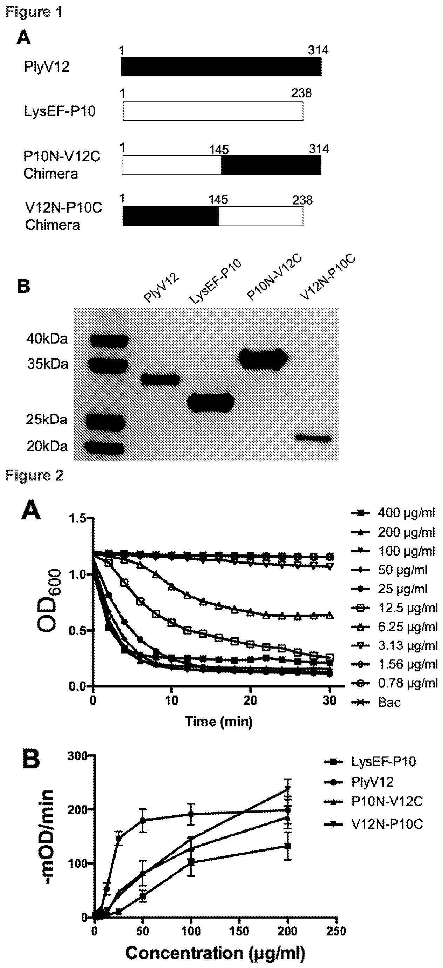

Two native lysins with different lytic spectra were selected as the parent lysins to generate chimeric lysins. LysEF-P10 is a narrow-spectrum lysin that only targets *E. faecalis* [Cheng, M. et al., Sci Rep 7: 1-15 (2017)], whereas PlyV12 is a broad-spectrum lysin that targets enterococci, staphylococci, and streptococci [Yoong, P. et al., J Bacteriol 186: 4808-4812 (2004)]. Two chimeric lysins, namely P10N-V12C and V12N-P10C, were created by swapping the CBD at the C-terminal domain of the two parent lysins (Fig. 1A). All 4 lysins were successfully expressed and purified (Fig. 1B). The protein bands corresponding to PlyV12 and V12N-P10C are slightly lower than their molecular weights, which is not unusual as such observations have been reported with other lysins [Guo, M. et al., Front Microbiol 8 (2017); Wittmann, J. et al., Microbiology 156: 2366-2373 (2010)]. It is worth noting that the high yield of PlyV12 lysin at 10 mg/L, is contrary to that previously reported [Yoong, P. et al., J Bacteriol 186: 4808-4812 (2004)]. The increased protein yield may be attributed to the recombinant gene with its codon usage optimized for *E. coli* expression.

### Example 3

### Dose response and killing kinetics of various lysins.

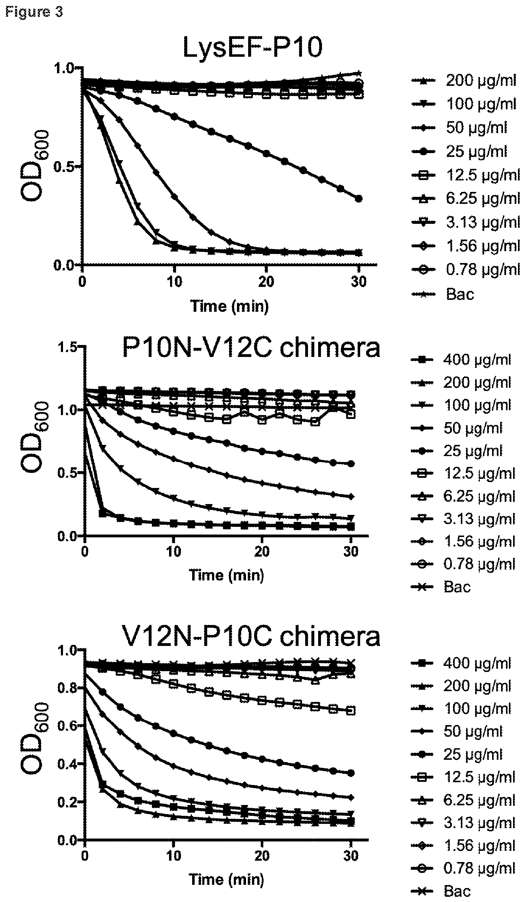

To determine the optimal doses of lysins for subsequent experiments, we measured the lytic activities of all 4 lysins against *E. faecalis* OG1RF strain by performing turbidity reduction assays. While PlyV12 requires at least 25 µg/ml to achieve >90% reduction in OD₆₀₀ in 30 minutes (Fig. 2A), the other 3 lysins require 100 µg/ml (Fig. 3). At these lysin concentrations, the lytic activities are optimal as indicated by their high lytic rates of over 100 -mOD/min (Fig. 2B). Therefore, 25 µg/ml for PlyV12 and 100 µg/ml for LysEF-P10, P10N-V12C and V12N-P10C were selected to further characterize the lysin.

### Example 4

### Lysis characterization at various pH and salt concentrations

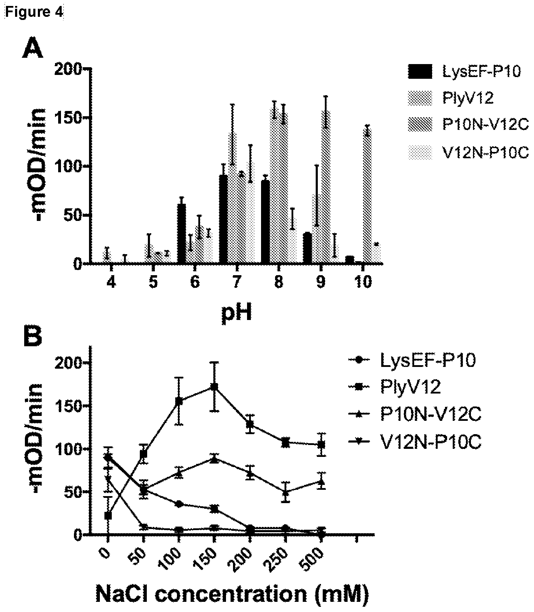

The lytic activities of lysins can vary significantly with pH and salinity [Cheng, M. et al., Sci Rep 7: 1-15 (2017); Yoong, P. et al., J Bacteriol 186: 4808-4812 (2004); Dong, Q. et al., Microb Biotechnol 8: 210-220 (2015); Swift, S. M. et al., FEMS Microbiol Lett 363: 1-8 (2016)]. Therefore, we tested the lysins against *E. faecalis* OG1RF strain in buffers of pH 4 to pH 10 and a series of salt concentrations ranging from 0 mM to 500 mM NaCl. We observed that P10N-V12C chimeric lysin maintains its lytic activity over a broader pH range, especially at pH 10, than the parent lysins, whereas LysEF-P10 and V12N-P10C share pH 7 as the optimal pH for lytic activity (Fig. 4A). When tested in buffer with increasing salt concentrations, the lytic activities of LysEF-P10 and V12N-P10C reduced significantly with negligible activity in buffers with above 200 mM NaCl (Fig. 4B). However, PlyV12 showed an opposite trend with low activity at 0 mM NaCl and maximum activity at 150 mM NaCl. Although the lytic activity of PlyV12 decreases slightly from 200 mM NaCl onwards, PlyV12 is still active at >100 -mOD/min. Interestingly, P10N-V12C chimera exhibit a superior salt tolerance than both of its parent lysins as it retains its lytic activity from 0 mM to 500 mM NaCl.

### Example 5

### Lytic spectra of lysins

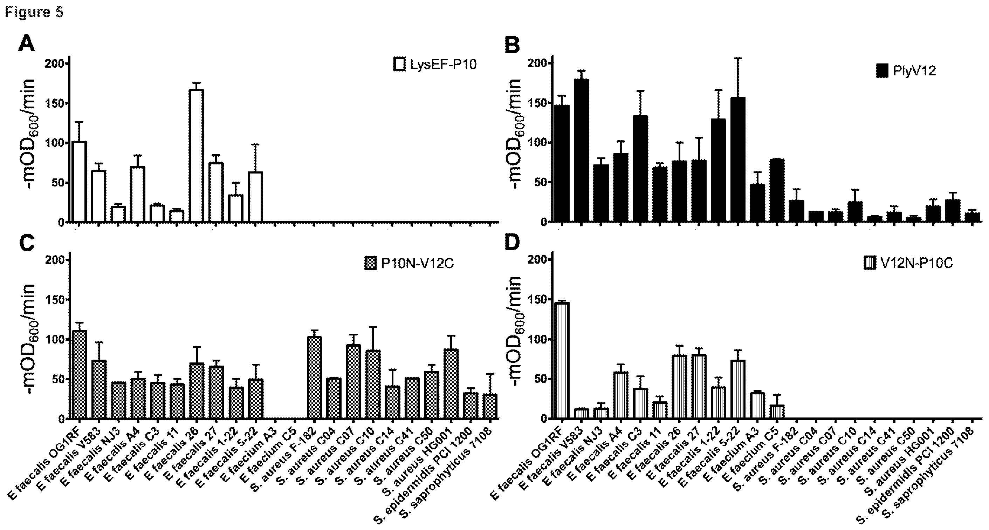

To determine whether the lytic spectrum of a lysin is dictated entirely by its CBD, we swapped the CBD of a species-specific lysin (P10C) with the CBD of a broad-spectrum lysin (V12C), and vice versa. The resulting chimeric lysins, P10N-V12C and V12N-P10C, along with the parent lysins, LysEF-P10 and PlyV12, were tested on 12 enterococcal and 10 staphylococcal strains, including 9 vancomycin-resistant enterococci (VRE) and 7 methicillin-resistance *Staphylococcus aureus* (MRSA) (Table 2). PlyV12 targets all enterococcal and staphylococcal strains (Fig. 5A). PlyV12 shows a slight preference towards enterococci than staphylococci as illustrated by the higher killing rate on enterococci, which is consistent with Yoong et al [J Bacteriol 186: 4808-4812 (2004)]. LysEF-P10 only targets the *E. faecalis* strains (Fig. 5B), with a lytic profile that is consistent with the previous study [Cheng, M. et al., Sci Rep 7: 1-15 (2017)].

**Table 2: Bacterial strains**

| Bacterial strains | Characteristics | Reference or source |
|---|---|---|
| Enterococci | | |
| *E. faecalis* OG1RF | Rifampicin resistant | ATCC 47077, * |
| *E. faecalis* V583 | Vancomycin resistant | ATCC 700802, ^ |
| *E. faecalis* NJ3 | Vancomycin resistant | ATCC 51299 |
| *E. faecalis* A4 | Isolate from healthy child gut | WUSTL, # |
| *E. faecalis* C3 | Isolate from healthy child gut | WUSTL, # |
| *E. faecalis* 11 | Non-VRE clinical wound isolate | TTSH |
| *E. faecalis 26* | Non-VRE clinical wound isolate | TTSH |
| *E. faecalis 27* | Non-VRE clinical wound isolate | TTSH |
| *E. faecalis* 1-22 | VRE clinical blood isolate | WUSTL, # |
| *E. faecalis* 5-22 | VRE clinical blood isolate | WUSTL, # |
| *E. faecium* A3 | Isolate from healthy child gut | WUSTL, # |
| *E. faecium* C5 | Isolate from healthy child gut | WUSTL, # |
| | | |

| Staphylococci | | |
|---|---|---|
| *S*. *aureus* F-182 | Methicillin resistant | ATCC 43300 |
| *S. aureus* C04 | MRSA clinical wound isolate | TTSH |
| *S. aureus* C07 | MRSA clinical wound isolate | TTSH |
| *S. aureus* C10 | MRSA clinical wound isolate | TTSH |
| *S. aureus* C14 | MRSA clinical wound isolate | TTSH |
| *S. aureus* C41 | MRSA clinical wound isolate | TTSH |
| *S. aureus* C50 | MRSA clinical wound isolate | TTSH |
| *S. aureus* HG001 | Methicillin susceptible | ! |
| *S. epidermidis* PCI 1200 | Non-pathogenic commensal | ATCC 12228 |
| *S. saprophyticus* 7108 | Wild-type strain | @ |
| *S. hominis* SK119 | Isolate from healthy skin | BEI resources |
| *S. hominis* C80 | Clinical isolate from sputum | BEI resources |

| | | |
|---|---|---|
| WUSTL: Washington University in St. Louis, USA. TTSH: Tan Tock Seng Hospital, Singapore. * Dunny, G. M. et al., Proc Natl Acad Sci 75:3479-3483 (1978) ^ Paulsen, I. T. et al., Science 299:2071-4 (2003) # Denno, D. M. et al., Clin Infect Dis 55:897-904 (2012) ! Caldelari, I. et al., Genome Announc 5 (2017) @ Gatemann, S. et al., FEMS Microbiol Lett 47:179-185 (1988) | | |

Swapping the broad-spectrum CBD from PlyV12 with a CBD from the narrow-spectrum LysEF-P10 lysin, the V12N-P10C chimera kills both *E. faecalis* and *E. faecium,* but it does not target staphylococci (Fig. 5D), suggesting the CBD is crucial in distinguishing bacteria of different genera. For the chimeric lysin with the CBD of PlyV12, P10N-V12C can target all staphylococcal strains and all *E. faecalis* strains (Fig. 5C). However, it does not kill *E. faecium.* This was surprising because a previously-reported chimeric lysin P187N-V12C, which also carries the CBD of PlyV12, was shown to lyse all of the tested streptococcal, staphylococcal, and enterococcal strains, including *E. faecium* [Dong, Q. et al., Microb Biotechnol 8: 210-220 (2015)]. Considering that the only difference between P187N-V12C and our P10N-V12C chimeric lysin is the catalytic domain, we deduced that the catalytic domain in P10N-V12C is responsible for distinguishing *E. faecium* from *E. faecalis.*

### Example 6

### The effect of P10N-V12C lysin on E. faecium

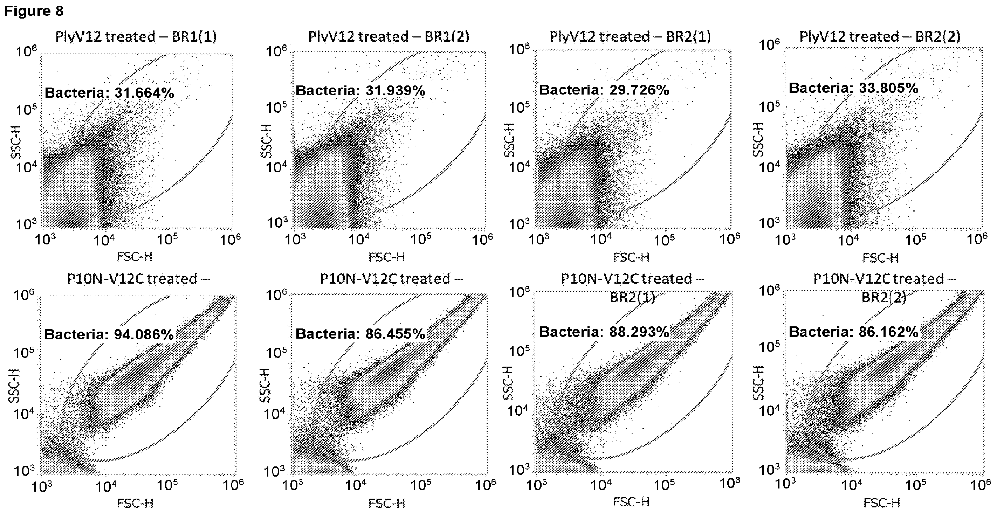

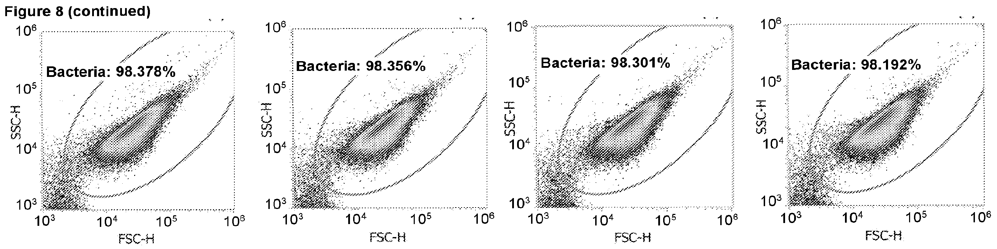

To investigate why P10N-V12C does not kill *E. faecium,* despite carrying a broad-spectrum CBD, lysin-treated *E. faecium* cells were further analyzed using flow cytometry and confocal microscopy. We first observed that PlyV12 effectively lysed most *E. faecium* cells as reflected by the low number of intact cells (Fig. 6A) and the 5-log CFU/ml reduction (Fig. 6G). The forward-scattered (FSC) and side-scattered (SSC) values of the PlyV12-treated cells (Fig. 6A) were drastically reduced compared to the untreated control (Fig. 6C). This reduction can be attributed to the cell debris and aggregates formed upon cell lysis. Additionally, significantly fewer bacterial cells were visible by confocal microscopy. Instead of individual intact cells, aggregates of presumably dead cells were observed upon PlyV12 treatment (Fig. 6H). By contrast, the P10N-V12C-treated *E. faecium* cells (Figs 6B and 8) were more elongated and slightly larger in overall size based on their FSC/SSC profiles compared to the untreated controls (Fig. 6C). These cells are still viable as the CFU count is similar to the control (Fig. 6G). However, most bacterial cells treated with P10N-V12C show significant uptake of PI dye as measured by flow cytometry (Fig. 6E) and observed in the confocal images (Fig. 6I), signifying the cell membrane has been compromised. The membrane is likely compromised by the lysins bound to the cell wall as the CBD of PlyV12 is known to bind to *E. faecium* [Dong, Q. et al., Microb Biotechnol 8: 210-220 (2015)]. Despite binding to the cell wall and compromising the membrane with its CBD, the chimeric lysin P10N-V12C does not lyse and kill *E. faecium* cells because its species-specific CD does not hydrolyze the peptidoglycan of *E. faecium.*

### Example 7

### Screening and isolation of chimeric lysins from a library

Further chimeric lysins were obtained by creating a library using Golden Gate assembly. Chimeric lysins generated with this method have a short linker peptide sequence between the lysin domains.

### Construction of lysin library

A library of 100 chimeric lysins was generated by shuffling the catalytic and cell-wall binding domains of 10 naturally occurring lysins using a cloning technique called Golden Gate assembly. The vector used is pNIC28-Bsa4 with Bsal restriction site that enables seamless cloning. The respective domains were identified by integrating the information from a list of sequence and structure prediction software, namely BLAST, JPRED, I-TASSER, and RaptorX. The nucleotide sequences of each domain were codon-optimized to improve the efficiency of soluble expression in *E. coli.* The amino acid and nucleotide sequences of the domains are represented by LKCHAPV12 (SEQ ID NOs: 9 and 10), V12NM3 (SEQ ID Nos: 13 and 14), LKNM3 (SEQ ID Nos: 15 and 16). The domains of each chimera were fused with a GSSG peptide linker. This GSSG linker was added between the two domains because it is a partial byproduct of Golden Gate assembly. Furthermore, the addition of a short linker has been previously shown to improve soluble expression as well as antibacterial activity [Yang, H. et al. Antimicrob. Agents Chemother. (2020) 64:e01610-19. doi: 10.1128/AAC.01610-19].

### Medium-throughput expression, screening and isolation

To screen the library for lysins with high activity against BO-causing bacteria, a medium-throughput protein expression method was developed with reference to Duyvejonck et al. (Duyvejonck L, et al., Antibiotics (Basel). (2021) 10(3):293. doi: 10.3390/antibiotics10030293). This method enables 200-400 variants to be tested at one time.

The protocol of the medium-throughput assay is as follows:
First, the gene library of 100 chimeric lysins was transformed in *E. coli* BL21 (DE3) T1R strain and spread on an LB agar plate with the selection antibiotics, namely kanamycin and chloramphenicol. Upon overnight incubation, the ~200 colonies were individually picked and inoculated into deep-well 96-well plates containing LB and the selection antibiotics for an overnight incubation with constant shaking at 700rpm. 10 µl of the overnight culture was used to inoculate another set of deep-well 96-well plates containing autoinduction media and the selection antibiotics, namely kanamycin and chloramphenicol. The 96-well plates were incubated at room temperature for 3 days to enable protein overexpression. The original 96-well plate with the uninduced overnight culture was made into glycerol stock and kept in - 80 °C for downstream isolation purpose. Upon the 3-day incubation, the plates were spun down to remove media and resuspended with 1x PBS. The expressed protein was obtained by lysing the cells using a freeze-thaw cycle method, namely putting the plate in liquid nitrogen and heated water bath for 7 times. The plates were spun down to remove the cell debris and the supernatant was transferred to another 96-well plate where it is ready to be tested for its activity.

The main difference in methodology is that we replaced the cell lysis method with a safer alternative. Duyvejonck et al. uses chloroform gas, a toxic and corrosive chemical, to lyse the cells whereas we use freeze-thaw method which uses liquid nitrogen and warm water to rapidly freeze and thaw the cells for 5-7 cycles, ultimately lysing the cells and releasing the intracellularly expressed protein to the supernatant.

Furthermore, we used different screening assays compared to Duyvejonck et al. We performed turbidity reduction assay and lysate clearance assay. The turbidity reduction assay is a routine assay to evaluate the activity of lysins targeting Gram-positive bacteria [Yoong, P. et al., J Bacteriol 186:4808-4812 (2004); Binte Muhammad Jai,H.S., et al., Front. Microbiol. 11: 2868 (2020)]. The lysate clearance assay was developed with reference to Raz et al [Raz A, et al., Antimicrob Agents Chemother. 2019 63(7):e00024-19. doi: 10.1128/AAC.00024-19], which was originally intended to test lysin's activity against Gramnegative bacteria. 3 bacterial species were tested on the turbidity reduction assay, namely *Staphylococcus hominis, Staphylococcus epidermidis,* and *Corynebacterium striatum.* For lysate clearance assay, only S. *epidermidis* was tested as it is an assay to test for catalytic activity as a proxy to gauge the expressed protein is functional.

For lysate clearance assay, 2.5 µl of lysate or purified proteins were spotted on the autoclaved bacterial agar of S. *epidermidis.* To prepare this bacterial agar, *S. epidermidis* was grown overnight in 1 L of LB, harvested and resuspended with 250 ml of 1x PBS. 1.75 g of agarose (0.7% agarose) was added to the resuspended solution before autoclaving and kept at 4 °C until use. The protocol of turbidity reduction assay is described in the next sections.

### Example 8

### Engineered chimeric lysins that can target body odor-causing bacteria

Table 3 shows a summary of the killing activities of 4 engineered lysins, LKCHAPV12, P10N-V12C, V12NM3 and LKNM3, when compared to PlyV12, the parent lysin that served as positive control. Lysin LKCHAPV12 exhibit good killing activity on all staphylococcal species tested and more importantly, LKCHAPV12 remains active in human sweat, whereas PlyV12 lost its activity entirely. Another 16 engineered lysins were tested but showed little activity in this assay (data not shown).

**Table 3: result table for engineered lysins #1-4 against positive control PlyV12**

| Lysins | Killing activity at 100 µg/ml | | | | | | | | | |
|---|---|---|---|---|---|---|---|---|---|---|
| | in PBS | | | | | | In sweat | | | |
| | *S. hominis* SK119 | | *S. epidermidis* | | *S. aureus* | | *S. hominis* | | *S. epidermidis* | |
| PlyV12 | 25 | ++ | 90 | +++ | 47 | ++ | 0 | - | 9 | + |
| LKCHAPV12 | 39 | ++ | 24.5 | ++ | 49.3 | ++ | 7.3 | + | 25.8 | ++ |
| P10N-V12C | 13.7 | ++ | 40 | ++ | 53.8 | +++ | 1.3 | + | 13.8 | ++ |
| V12NM3 | 5.8 | + | 27.3 | ++ | 25 | ++ | 0 | - | 0 | - |
| LKNM3 | 7.3 | + | 15.5 | ++ | 22.5 | ++ | 0 | - | 2.3 | + |

| | | | | | | | | | | |
|---|---|---|---|---|---|---|---|---|---|---|
| The activity levels of the lysins against various bacteria at 100 µg/ml are also shown as -, +, ++ +++ - refers to no killing + refers to -mOD/min below 10 ++ refers to -mOD/min between 10 and 50 +++ refers to -mOD/min above 50 | | | | | | | | | | |

Additionally, P10N-V12C also remains active against *S. hominis* in human sweat. 4 out of the 5 lysins tested are active against *S*. *epidermidis* in human sweat while only LKCHAPV12 and P10N-V12C are active against *S. hominis* in human sweat, suggesting *S. hominis* is harder to kill in human sweat. Another 2 engineered lysins, V12NM3 and LKNM3, are active against *S*. *hominis, S. epidermidis,* and *S. aureus* in PBS but mostly lost their activity when they are in the sweat environment.

### Example 9

### Combination of lysins can achieve enhanced bacterial killing

Enhanced killing was achieved when combining an engineered lysin (LKCHAPV12) and a native lysin (PlyV12) (Table 4).

**Table 4: result table illustrating synergistic activity between PlyV12 and LKCHAPV12 on Staphylococcus hominis.**

| **% of OD reduction after 1 hour treatment in turbidity reduction assay** | | | | | | | | | | |
|---|---|---|---|---|---|---|---|---|---|---|
| **PlyV12 + LKCHAPV12** | **PlyV12 (µg/ml)** | **1** | **2** | **3** | **4** | **5** | **6** | **7** | **8** | |
| **A** | **100** | 21 | 68 | 86 | 93 | 91 | 82 | 77 | 64 | |
| **B** | **50** | 0 | 73 | 86 | 92 | 90 | 86 | 77 | 72 | |
| **C** | **25** | 0 | 79 | 87 | 86 | 91 | 88 | 76 | 71 | |
| **D** | **12.5** | 0 | 54 | 34 | 88 | 92 | 90 | 80 | 70 | |
| **E** | **6.25** | 0 | 24 | 75 | 81 | 95 | 90 | 83 | 77 | |
| **F** | **3.125** | 0 | 13 | 51 | 73 | 88 | 85 | 79 | 78 | |
| **G** | **1.5625** | 0 | 8 | 28 | 63 | 86 | 82 | 80 | 70 | |
| **H** | **0** | 0 | 0 | 28 | 53 | 69 | 73 | 57 | 69 | |
| | | **0** | **1.6** | **3.125** | **6.25** | **12.5** | **25** | **50** | **100** | **LKCHAPV12 (µg/ml)** |

Notably, >80% killing can be achieved by combining 6.25 µg/ml of PlyV12 and LKCHAPV12, respectively, whereas it would require >100 µg/ml of each lysin to achieve individually. Such synergistic effect will lower the effective materials needed, thus translating to cost reduction. The apparent lower percentage of OD reduction at higher concentrations (50-100 µg/ml) is a result of formation of cell clumps. The cultures in those wells were clear, indicating >85% cell lysis and death.

### Summary

With the emergence of multidrug-resistant bacteria, including VRE and MRSA, there is an urgent need to develop new antibacterial therapeutics.

In this study, we constructed chimeric lysins by swapping the CBD of two parent lysins with different properties. We found that domain-swapping affects the functional pH and salinity tolerance of the two chimeric lysins. P10N-V12C outperformed its parent lysins by maintaining high lytic activities across a broader range of pH and salt concentrations. However, the other chimeric lysin, V12N-P10C, has limited pH range with diminished activity in the presence of salt. These observations show the importance of CBD in determining the optimal pH and salt concentration where a lysin is most active at. As observed in lysin LKCHAPV12, the chimeric lysin showed superior activity in human sweat compared to its parent lysin PlyV12, thereby illustrating the advantages of chimeric lysins.

The two parent lysins selected in this study have different lytic spectra: LysEF-P10 only targeting *E. faecalis* and PlyV12 targeting multiple genera. Upon swapping the CBD of LysEF-P10, the lytic spectrum of V12N-P10C was narrowed as it only targets enterococci. Conversely, P10N-V12C exhibits a broadened lytic spectrum with the CBD of PlyV12. Interestingly, although P10N-V12C now targets staphylococcal strains, it retains the species-specificity feature of LysEF-P10 on *E. faecalis* and does not target *E. faecium.* These data suggest that the CD also contributes, albeit to a lesser extent compared to the CBD, to the final lytic spectrum of a chimeric lysin.

Flow cytometry and confocal microscopy revealed that the *E. faecium* cells that have been treated with P10N-V12C have a compromised membrane, but the cells remain intact and viable. It was previously shown that GFP-fused CBD of PlyV12 binds to staphylococcal and enterococcal cells, including *E. faecium* [Dong, Q. et al., Microb Biotechnol 8:210-220 (2015)], therefore the CBD of P10N-V12C is expected to bind to the membrane of *E*. *faecium.* This could explain the slightly larger and elongated *E. faecium* cells upon exposure of P10N-V12C. Such binding event would disrupt the bacterial membrane to an extent that enables the PI uptake of the cells. However, the treated cells were not lysed and remained viable as the CD of P10N-V12C does not hydrolyze the peptidoglycan of *E. faecium.*

The present disclosure provides species specificity to an otherwise, broad-spectrum lysin by swapping its catalytic domain with that of a species-specific lysin. The resulting chimeric lysin P10N-V12C selectively targets *E. faecalis* over *E. faecium* while it remains active against all staphylococcal strains tested. It possesses a high lytic activity across a broader range of pH and salt concentrations than any of the parent lysins. While CBD swapping helps to extend the lytic spectrum such that multiple bacterial genera can be targeted, choosing the correct CD to fuse with the CBD is important to achieve species specificity in a particular genus. With more understanding of the roles of both CD and CBD, novel lysins can be tailor-made to develop a highly-targeted antimicrobial therapy.

Not every chimeric lysin is suitable to be used for commercial applications. Out of the 100 possible chimeric lysins we screened, some lost their activity in moderate-to-high salinity environment, and some did not have soluble expression. Here we have identified 4 engineered lysins with the potential to be used in treating body odor, atopic dermatitis (eczema), bacteremia, as well as healing wound with bacterial infections. These lysins can be used alone, or in combination to achieve additive or synergistic effect. For example, PlyV12 and LKCHAPV12 exhibit synergy in killing Staphylococcal when both are used in combination.

### References

1. Binte Muhammad Jai,H.S., Dam,L.C., Tay,L.S., Koh,J.J.W., Loo,H.L., Kline,K.A. and Goh,B.C. (2020) Engineered Lysins With Customized Lytic Activities Against Enterococci and Staphylococci. Front. Microbiol., 11: 2868.
2. Broendum, S. S., Buckle, A. M. & McGowan, S. Catalytic diversity and cell wall binding repeats in the phage-encoded endolysins. Mol. Microbiol. 110, 879-896 (2018).
3. Caldelari, I. et al. Complete Genome Sequence and Annotation of the Staphylococcus aureus Strain HG001. Genome Announc. 5, (2017).
4. Cheng, M. et al. Endolysin LysEF-P10 shows potential as an alternative treatment strategy for multidrug-resistant Enterococcus faecalis infections. Sci. Rep. 7, 1-15 (2017).
5. Denno, D. M. et al. Diarrhea Etiology in a Pediatric Emergency Department: A Case Control Study. Clin. Infect. Dis. 55, 897-904 (2012).
6. Deutsch, S.-M., Guezenec, S., Piot, M., Foster, S. & Lortal, S. Mur-LH, the Broad-Spectrum Endolysin of Lactobacillus helveticus Temperate Bacteriophage φ-0303. Appl. Environ. Microbiol. 70, 96-103 (2004).
7. Diez-Martinez, R. et al. Improving the Lethal Effect of Cpl-7, a Pneumococcal Phage Lysozyme with Broad Bactericidal Activity, by Inverting the Net Charge of Its Cell Wall-Binding Module. Antimicrob. Agents Chemother. 57, 5355-5365 (2013).
8. Dong, Q. et al. Construction of a chimeric lysin Ply187N-V12C with extended lytic activity against staphylococci and streptococci. Microb. Biotechnol. 8, 210-220 (2015).
9. Dunny, G. M., Brown, B. L. & Clewell, D. B. Induced cell aggregation and mating in Streptococcus faecalis: evidence for a bacterial sex pheromone. Proc. Natl. Acad. Sci. 75, 3479-3483 (1978).
10. Duyvejonck L, Gerstmans H, Stock M, Grimon D, Lavigne R, Briers Y. Rapid and High-Throughput Evaluation of Diverse Configurations of Engineered Lysins Using the VersaTile Technique. Antibiotics (Basel). 2021 Mar 11;10(3):293. doi: 10.3390/antibiotics10030293.
11. Fischetti, V. A. Bacteriophage lysins as effective antibacterials. Curr. Opin. Microbiol. 11, 393-400 (2008).
12. Fischetti, V. A. Bacteriophage endolysins: A novel anti-infective to control Gram-positive pathogens. Int. J. Med. Microbiol. 300, 357-362 (2010).
13. Fischetti, V. A. Development of phage lysins as novel therapeutics: A historical perspective. Viruses 10, (2018).
14. Gatermann, S., Marre, R., Heesemannn, J. & Henkel, W. Hemagglutinating and adherence properties of Staphylococcus saprophyticus : epidemiology and virulence in experimental urinary tract infection of rats. FEMS Microbiol. Lett. 47, 179-185 (1988).
15. Gilmer, D. B., Schmitz, J. E., Euler, C. W. & Fischetti, V. A. Novel Bacteriophage Lysin with Broad Lytic Activity Protects against Mixed Infection by Streptococcus pyogenes and Methicillin-Resistant Staphylococcus aureus. Antimicrob. Agents Chemother. 57, 2743-2750 (2013).
16. Guo, M. et al. A Novel Antimicrobial Endolysin, LysPA26, against Pseudomonas aeruginosa. Front. Microbiol. 8, (2017).
17. Hermoso, J. A., Garcia, J. L. & Garcia, P. Taking aim on bacterial pathogens: from phage therapy to enzybiotics. Curr. Opin. Microbiol. 10, 461-472 (2007).
18. Loessner, M. J., Kramer, K., Ebel, F. & Scherer, S. C-terminal domains of Listeria monocytogenes bacteriophage murein hydrolases determine specific recognition and high-affinity binding to bacterial cell wall carbohydrates. Mol. Microbiol. 44, 335-349 (2002).
19. López, R., Garcia, E., Garcia, P. & Garcia, J. L. The Pneumococcal Cell Wall Degrading Enzymes: A Modular Design to Create New Lysins? Microb. Drug Resist. 3, 199-211 (1997).
20. Love, M. J., Abeysekera, G. S., Muscroft-Taylor, A. C., Billington, C. & Dobson, R. C. J. On the catalytic mechanism of bacteriophage endolysins: Opportunities for engineering. Biochim. Biophys. Acta - Proteins Proteomics 1868, 140302 (2020).
21. Low, L. Y., Yang, C., Perego, M., Osterman, A. & Liddington, R. Role of net charge on catalytic domain and influence of cell wall binding domain on bactericidal activity, specificity, and host range of phage lysins. J. Biol. Chem. 286, 34391-34403 (2011).
22. Maciejewska, B., Olszak, T. & Drulis-Kawa, Z. Applications of bacteriophages versus phage enzymes to combat and cure bacterial infections: an ambitious and also a realistic application? Appl. Microbiol. Biotechnol. 102, 2563-2581 (2018).
23. Nelson, D., Loomis, L. & Fischetti, V. A. Prevention and elimination of upper respiratory colonization of mice by group A streptococci by using a bacteriophage lytic enzyme. Proc. Natl. Acad. Sci. 98, 4107-4112 (2001).
24. Nelson, D. C. et al. Endolysins as Antimicrobials. in Advances in Virus Research (2012). doi:10.1016/B978-0-12-394438-2.00007-4
25. O'Flaherty, S., Coffey, A., Meaney, W., Fitzgerald, G. F. & Ross, R. P. The Recombinant Phage Lysin LysK Has a Broad Spectrum of Lytic Activity against Clinically Relevant Staphylococci, Including Methicillin-Resistant Staphylococcus aureus. J. Bacteriol. 187, 7161 - 7164 (2005).
26. Oliveira, H. et al. Molecular Aspects and Comparative Genomics of Bacteriophage Endolysins. J. Virol. 87, 4558-4570 (2013).
27. Oliveira, H., São-José, C. & Azeredo, J. Phage-derived peptidoglycan degrading enzymes: Challenges and future prospects for in vivo therapy. Viruses 10, (2018).
28. Paulsen, I. T. et al. Role of mobile DNA in the evolution of vancomycin-resistant Enterococcus faecalis. Science 299, 2071-4 (2003).
29. Raz A, Serrano A, Hernandez A, Euler CW, Fischetti VA. Isolation of Phage Lysins That Effectively Kill Pseudomonas aeruginosa in Mouse Models of Lung and Skin Infection. Antimicrob Agents Chemother. 2019 Jun 24;63(7):e00024-19. doi: 10.1128/AAC.00024-19.
30. Schleifer, K. H. & Kandler, O. Peptidoglycan types of bacterial cell walls and their taxonomic implications. Bacteriol. Rev. 36, 407-77 (1972).
31. Schmelcher, M., Donovan, D. M. & Loessner, M. J. Bacteriophage endolysins as novel antimicrobials. Future Microbiol. 7, 1147-1171 (2012).
32. Schmelcher, M., Tchang, V. S. & Loessner, M. J. Domain shuffling and module engineering of Listeria phage endolysins for enhanced lytic activity and binding affinity. Microb. Biotechnol. 4, 651-662 (2011).
33. Swift, S. M. et al. The endolysin from the Enterococcus faecalis bacteriophage VD13 and conditions stimulating its lytic activity. FEMS Microbiol. Lett. 363, 1-8 (2016).
34. Velez, R. & Sloand, E. Combating antibiotic resistance, mitigating future threats and ongoing initiatives. J. Clin. Nurs. 25, 1886-1889 (2016).
35. Ventola, C. L. The antibiotic resistance crisis: part 1: causes and threats. P T 40, 277-83 (2015).
36. Vermassen, A. et al. Cell wall hydrolases in bacteria: Insight on the diversity of cell wall amidases, glycosidases and peptidases toward peptidoglycan. Front. Microbiol. 10, (2019).
37. Wittmann, J., Eichenlaub, R. & Dreiseikelmann, B. The endolysins of bacteriophages CMP1 and CN77 are specific for the lysis of Clavibacter michiganensis strains. Microbiology 156, 2366-2373 (2010).
38. Yang, H., Yu, J. & Wei, H. Engineered bacteriophage lysins as novel anti-infectives. Front. Microbiol. 5, 1-6 (2014).
39. Yang, H. et al. Novel Chimeric Lysin with High-Level Antimicrobial Activity against Methicillin-Resistant Staphylococcus aureus In Vitro and In Vivo. Antimicrob. Agents Chemother. 58, 536-542 (2014).
40. Yang, H., Zhang, H., Wang, J., Yu, J. & Wei, H. A novel chimeric lysin with robust antibacterial activity against planktonic and biofilm methicillin-resistant Staphylococcus aureus. Sci. Rep. 7, 1-13 (2017).
41. Yang, H. et al. ClyJ Is a Novel Pneumococcal Chimeric Lysin with a Cysteine- and Histidine-Dependent Amidohydrolase/Peptidase Catalytic Domain. Antimicrob. Agents Chemother. (2019) 63:e02043-18. doi: 10.1128/AAC.02043-18.
42. Yang, H. et al. Linker Editing of Pneumococcal Lysin ClyJ Conveys Improved Bactericidal Activity. Antimicrob. Agents Chemother. (2020) 64, :e01610-19. doi: 10.1128/AAC.01610-19.
43. Yoong, P., Schuch, R., Nelson, D. & Fischetti, V. A. Identification of a Broadly Active Phage Lytic Enzyme with Lethal Activity against Antibiotic-Resistant Enterococcus faecalis and Enterococcus faecium. J. Bacteriol. 186, 4808-4812 (2004).
44. Zimmer, M., Sattelberger, E., Inman, R. B., Calendar, R. & Loessner, M. J. Genome and proteome of Listeria monocytogenes phage PSA: an unusual case for programmed + 1 translational frameshifting in structural protein synthesis. Mol. Microbiol. 50, 303-317 (2003).
45. Zou, G. et al. Functional Analysis of Two Cavities in Flavivirus NS5 Polymerase. J. Biol. Chem. 286, 14362-14372 (2011).

## Claims

1. A chimeric bacteriophage lysin comprising a catalytic domain of a first phage lysin and a binding domain of a second phage lysin, wherein the chimeric bacteriophage lysin has killing activity against a plurality of *Staphylococcus* species, wherein the chimeric bacteriophage lysin comprises an amino acid sequence set forth in:
amino acids 1-165 and 170-338 set forth in SEQ ID NO: 9, or
a variant having at least 90% sequence identity to the amino acid sequence set forth in SEQ ID NO: 9,
and wherein the chimeric bacteriophage lysin has killing activity against *S*. *hominis* and *S. epidermidis* in human sweat.

2. The chimeric bacteriophage lysin of claim 1, wherein the variant comprises a sequence having at least 95% sequence identity to the amino acid sequence set forth in SEQ ID NO: 9.

3. The chimeric bacteriophage lysin of claim 1 or 2, wherein the said catalytic domain of a first phage lysin and binding domain of a second phage lysin are fused by a linker peptide.

4. The chimeric bacteriophage lysin of claim 3, wherein the linker peptide has the amino acid sequence set forth in amino acids 166-169 of SEQ ID NO: 9.

5. The chimeric bacteriophage lysin of any one of claims 1 to 4, wherein the chimeric bacteriophage lysin has the amino acid sequence set forth in SEQ ID NO: 9.

6. An isolated polynucleotide molecule encoding a chimeric bacteriophage lysin defined in any one of claims 1 to 5.

7. The isolated polynucleotide molecule of claim 6, wherein the nucleic acid sequence has at least 85% sequence identity to SEQ ID NO: 10 due to the degeneracy of the genetic code.

8. A composition comprising one or more chimeric bacteriophage lysins of any one of claims 1 to 7.

9. The composition of claim 8, further comprising an antibiotic and/or a native lysin such as PlyV12 (SEQ ID NO: 17) or LysEF-P10 (SEQ ID NO: 19).

10. The composition of claim 9, wherein the native lysin is PlyV12 (SEQ ID NO: 17).

11. A cosmetic use of a composition of any one of claims 8 to 10 for the prevention or reduction of body odor.

12. The cosmetic use of claim 11, wherein the use comprises a reduction or prevention of growth of a *staphylococcus* species.

13. Use of a composition of any one of claims 8 to 10 for sanitizing or decontaminating porous or non-porous surfaces.

14. A method for decontaminating inanimate surfaces suspected of containing infectious bacteria, comprising treatment of said surfaces with a bactericidally or bacteriostatically effective amount of at least one chimeric bacteriophage lysin of any one of claims 1 to 5 or the composition of claim 8 to 10.

15. The chimeric bacteriophage lysin of any one of claims 1 to 5 or the composition of claim 8 to 10 for use in medicine.

## Patentansprüche

1. Chimäres Bakteriophagen-Lysin, das eine katalytische Domäne eines ersten Phagen-Lysins und eine Bindungsdomäne eines zweiten Phagen-Lysins umfasst, wobei das chimäre Bakteriophagen-Lysin abtötende Wirkung gegen eine Vielzahl von Staphylococcus-Spezies aufweist, wobei das chimäre Bakteriophagen-Lysin eine Aminosäuresequenz umfasst, die in den folgenden dargelegt ist:
in den Aminosäuren 1-165 und 170-380 wie in SEQ ID NO: 9 dargelegt oder
in einer Variante mit zumindest 90 % Sequenzidentität mit der in SEQ ID NO: 9 dargelegten Aminosäuresequenz,
und wobei das chimäre Bakteriophagen-Lysin abtötende Wirkung gegen S. hominis und S. epidermidis in menschlichem Schweiß aufweist.

2. Chimäres Bakteriophagen-Lysin nach Anspruch 1, wobei die Variante eine Sequenz mit zumindest 95 % Sequenzidentität mit der in SEQ ID NO: 9 dargelegten Aminosäuresequenz aufweist.

3. Chimäres Bakteriophagen-Lysin nach Anspruch 1 oder 2, wobei die katalytische Domäne eines ersten Phagen-Lysins und die Bindungsdomäne eines zweiten Phagen-Lysins über ein Linker-Peptid verbunden sind.

4. Chimäres Bakteriophagen-Lysin nach Anspruch 3, wobei das Linker-Peptid die in den Aminosäuren 166-169 von SEQ ID NO: 9 dargelegte Aminosäuresequenz aufweist.

5. Chimäres Bakteriophagen-Lysin nach einem der Ansprüche 1 bis 4, wobei das chimäre Bakteriophagen-Lysin die in SEQ ID NO: 9 dargelegte Aminosäuresequenz aufweist.

6. Isoliertes Polynukleotid-Molekül, das für ein chimäres Bakteriophagen-Lysin gemäß einem der Ansprüche 1 bis 5 kodiert.

7. Isoliertes Polynukleotid-Molekül nach Anspruch 6, wobei die Nukleinsäuresequenz aufgrund der Degeneriertheit des genetischen Codes zumindest 85 % Sequenzidentität mit SEQ ID NO: 10 aufweist.

8. Zusammensetzung, die ein oder mehrere chimäre Bakteriophagen-Lysine nach einem der Ansprüche 1 bis 7 umfasst.

9. Zusammensetzung nach Anspruch 8, die weiters ein Antibiotikum und/oder ein natives Lysin wie PlyV12 (SEQ ID NO: 17) oder LysEF-P10 (SEQ ID NO: 19) umfasst.

10. Zusammensetzung nach Anspruch 9, wobei das native Lysin PlyV12 (SEQ ID NO: 17) ist.

11. Kosmetische Verwendung einer Zusammensetzung nach einem der Ansprüche 8 bis 10 zur Vorbeugung gegen oder Verringerung von Körpergeruch.

12. Kosmetische Verwendung nach Anspruch 11, wobei die Verwendung die Verringerung des Wachstums oder die Vorbeugung gegen das Wachstum einer Staphylococcus-Spezies umfasst.

13. Verwendung einer Zusammensetzung nach einem der Ansprüche 8 bis 10 zur Desinfektion oder Dekontamination von porösen oder nichtporösen Oberflächen.

14. Verfahren zur Dekontamination von unbelebten Oberflächen, von denen befürchtet wird, dass sie infektiöse Bakterien aufweisen, welches die Behandlung der Oberflächen mit einer bakterizid oder bakteriostatisch wirksamen Menge zumindest eines chimären Bakteriophagen-Lysins nach einem der Ansprüche 1 bis 5 oder einer Zusammensetzung nach Anspruch 8 bis 10 umfasst.

15. Chimäres Bakteriophagen-Lysin nach einem der Ansprüche 1 bis 5 oder Zusammensetzung nach Anspruch 8 bis 10 zur Verwendung in der Medizin.

## Revendications

1. Lysine de bactériophage chimérique comprenant un domaine catalytique d'une première lysine de phage et un domaine de liaison d'une seconde lysine de phage, dans laquelle la lysine de bactériophage chimérique présente une activité lytique contre une pluralité d'espèces de *Staphylococcus,* dans laquelle la lysine de bactériophage chimérique comprend une séquence d'acides aminés définie :
acides aminés 1-165 et 170-338 définis SEQ ID NO: 9, ou
une variante présentant au moins 90 % d'identité de séquence avec la séquence d'acides aminés définie dans SEQ ID NO: 9,
et dans laquelle la lysine de bactériophage chimérique présente une activité lytique contre *S. hominis* et *S. epidermidis* dans la sueur humaine.

2. Lysine de bactériophage chimérique selon la revendication 1, dans laquelle la variante comprend une séquence présentant au moins 95 % d'identité de séquence avec la séquence d'acides aminés définie dans SEQ ID NO: 9.

3. Lysine de bactériophage chimérique selon la revendication 1 ou 2, dans laquelle ledit domaine catalytique de la première lysine de phage et ledit domaine de liaison de la seconde lysine de phage sont fusionnés par un peptide lieur.

4. Lysine de bactériophage chimérique selon la revendication 3, dans laquelle le peptide lieur présente la séquence d'acides aminés correspondant aux acides aminés 166-169 de SEQ ID NO: 9.

5. Lysine de bactériophage chimérique selon l'une quelconque des revendications 1 à 4, dans laquelle la lysine de bactériophage chimérique présente la séquence d'acides aminés définie dans SEQ ID NO: 9.

6. Molécule polynucléotidique isolée codant une lysine de bactériophage chimérique telle que définie dans l'une quelconque des revendications 1 à 5.

7. Molécule polynucléotidique isolée selon la revendication 6, dans laquelle la séquence d'acide nucléique présente au moins 85 % d'identité de séquence avec SEQ ID NO: 10 en raison de la dégénérescence du code génétique.

8. Composition comprenant une ou plusieurs lysines de bactériophage chimériques selon l'une quelconque des revendications 1 à 7.

9. Composition selon la revendication 8, comprenant en outre un antibiotique et/ou une lysine native telle que PlyV12 (SEQ ID NO: 17) ou LysEF-P10 (SEQ ID NO: 19).

10. Composition selon la revendication 9, dans laquelle la lysine native est PlyV12 (SEQ ID NO: 17).

11. Utilisation cosmétique d'une composition selon l'une quelconque des revendications 8 à 10 pour la prévention ou la réduction des odeurs corporelles.

12. Utilisation cosmétique selon la revendication 11, dans laquelle l'utilisation comprend une réduction ou une prévention de la croissance d'une espèce de *Staphylococcus.*

13. Utilisation d'une composition selon l'une quelconque des revendications 8 à 10 pour l'assainissement ou la décontamination de surfaces poreuses ou non poreuses.

14. Procédé de décontamination de surfaces inanimées suspectées de contenir des bactéries infectieuses, comprenant le traitement desdites surfaces avec une quantité, bactéricidement ou bactériostatiquement efficace, d'au moins une lysine de bactériophage chimérique selon l'une quelconque des revendications 1 à 5 ou de la composition selon l'une quelconque des revendications 8 à 10.

15. Lysine de bactériophage chimérique selon l'une quelconque des revendications 1 à 5 ou composition selon l'une quelconque des revendications 8 à 10, pour utilisation en médecine.
